(19) Europäisches Patentamt
European Patent Office
Office européen des brevets

(11) **EP 3 738 945 B1**

(12) **EUROPÄISCHE PATENTSCHRIFT**

(45) Veröffentlichungstag und Bekanntmachung des Hinweises auf die Patenterteilung:
**12.07.2023   Patentblatt 2023/28**

(21) Anmeldenummer: **20174408.3**

(22) Anmeldetag: **13.05.2020**

(51) Internationale Patentklassifikation (IPC):
***C07C 2/10*** *(2006.01)*   ***C07C 11/02*** *(2006.01)*
***C10G 50/00*** *(2006.01)*

(52) Gemeinsame Patentklassifikation (CPC):
(C-Sets verfügbar)
**C07C 2/10; C10G 50/00;** C07C 2521/12;
C07C 2523/755                    (Forts.)

(54) **VERFAHREN ZUR INHIBIERUNG EINER OLIGOMERISIERUNG VON C3- BIS C5-OLEFINEN**

METHOD FOR INHIBITING AN OLIGOMERIZATION OF C3 TO C5 OLEFINS

PROCÉDÉ D'INHIBITION D'UNE OLIGOMÉRISATION D'OLÉFINES C3 À C5

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.05.2019   EP 19174290**

(43) Veröffentlichungstag der Anmeldung:
**18.11.2020   Patentblatt 2020/47**

(73) Patentinhaber: **Evonik Operations GmbH**
**45128 Essen (DE)**

(72) Erfinder:
• **Peitz, Stephan**
  **45739 Oer-Erkenschwick (DE)**
• **Stochniol, Guido**
  **45721 Haltern am See (DE)**
• **Bukohl, Reiner**
  **45770 Marl (DE)**
• **Reeker, Helene**
  **44227 Dortmund (DE)**
• **Paul, Niklas**
  **45770 Marl (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A2- 1 457 475    WO-A1-2014/207034**

**(Forts. nächste Seite)**

(52) Gemeinsame Patentklassifikation (CPC): (Forts.)

    C-Sets
    **C07C 2/10, C07C 11/02**

**Beschreibung**

Die vorliegende Erfindung betrifft ein Verfahren zur Inhibierung einer Oligomerisierung von C3- bis C5-Olefinen unter Verwendung eines Katalysators, wobei die Oligomerisierung in mindestens einer Reaktionsstufe, die mindestens einen Reaktor und mindestens eine Destillationskolonne umfasst, durchgeführt wird und wobei der Gehalt an Oligomeren im Zulaufstrom zu dem mindestens einen Reaktor der mindestens einen Reaktionsstufe mindestens 1 Gew.-% ist.

Generell versteht man unter der Oligomerisierung die Reaktion von ungesättigten Kohlenwasserstoffen mit sich selbst, wobei entsprechend längerkettige Kohlenwasserstoffe, die so genannten Oligomere, entstehen. So lässt sich beispielsweise durch die Oligomerisierung von zwei Olefinen mit drei Kohlenstoffatomen (Propen) ein Olefin mit sechs Kohlenstoffatomen (Hexen) aufbauen. Die Oligomerisierung von zwei Molekülen miteinander wird auch Dimerisierung genannt.

Die erhaltenen Oligomere sind Zwischenprodukte, die beispielsweise für die Herstellung von Aldehyden, Carbonsäuren und Alkoholen eingesetzt werden. Die Oligomerisierung von Olefinen wird großtechnisch entweder in homogener Phase an einem gelösten oder heterogen an einem festen Katalysator oder mit einem zweiphasigen Katalysatorsystem durchgeführt.

Verfahren zur Oligomerisierung von Olefinen sind im Stand der Technik hinlänglich bekannt und werden großindustriell eingesetzt. Die Produktionsmengen belaufen sich alleine in Deutschland auf mehrere tausend Kilotonnen pro Jahr. Die Quelle für die Olefine für die Oligomerisierungsverfahren sind in der Regel olefinhaltige Fraktionen aus Crackverfahren (beispielweise Steamcracker oder Fluidkatalystische Cracker), die neben den Olefinen auch entsprechende Alkane enthalten.

Ein bekanntes Verfahren zur Oligomerisierung wird in der WO2014/207034 A1 beschrieben. Das dort offenbarte Verfahren betrifft eine Oligomerisierung von C4-Strömen mit geringen 1-Buten Gehalten, was auch als "OCTOL-Prozess" bekannt ist. Die Oligomerisierung wird auf heterogenen Nickel-enthaltenden Katalysatoren durchgeführt.

Ein weiteres bekanntes Verfahren zur Herstellung von Oligomeren aus Alkenen mit 4 bis 8 Kohlenstoffen ist in der EP 1 457 475 A2 offenbart. Dabei sollte durch eine verbesserte Verwendung des Feeds eine verlängerte Katalysatorstandzeit erreicht werden können. Als geeignete Katalysatoren werden Nickel-enthaltende Katalysatoren offenbart.

Bei technischen Oligomerisierungsverfahren kann es während des Betriebs aufgrund verschiedener ggf. sich bedingender Effekte zu hohen Reaktionsraten der beteiligten Olefine kommen. Diese hohen Reaktionsraten können über den gesamten Reaktor oder auch nur lokal, das heißt an räumlich begrenzten Orten im Reaktor auftreten. Da die Oligomerisierungsreaktion eine exotherme Reaktion ist, können zu hohe Reaktionsraten auch zu einer sich selbst beschleunigenden Reaktion führen, die mit einer unerwünschten Nebenproduktbildung, dem Auftreten von Cracking-Reaktionen, einer Zerstörung des Katalysators, einer erhöhten Wärmeentwicklung (lokal oder über den gesamten Reaktor) und unzulässigen Überdrücken bis zum Produktaustritt einhergehen können.

Die Aufgabe der vorliegenden Erfindung war deshalb die Bereitstellung eines Verfahrens mit dem eine Oligomerisierungsreaktion von C3- bis C5-Olefinen gezielt inhibiert werden kann, um negativen Auswirkungen von ggf. lokal erhöhten Reaktionsraten zu minimieren oder ganz zu unterbinden.

Es konnte überraschenderweise herausgefunden werden, dass die beschriebenen negativen Effekte (Anstieg der Reaktionsrate, erhöhte Wärmeentwicklung, erhöhte Nebenproduktbildung) gerade bei einer ausreichenden Abtrennung der Oligomere vom restlichen Oligomerisat, welches zum Reaktor zurückgeführt wird, auftreten. Wird dagegen der Gehalt an Oligomeren im Zulauf zu dem mindestens einem Reaktor der mindestens einen Reaktionsstufe, welcher aus dem zurückgeführten restlichen Oligomerisat und dem Frischzulauf des eingesetzten Einsatzgemisches besteht, erhöht, treten die negative Effekte nicht auf oder können verringert werden. Die zugrundeliegende Aufgabe konnte gemäß der vorliegenden Erfindung deshalb dadurch gelöst werden, dass der Gehalt an Oligomeren im Zulauf zu dem mindestens einem Reaktor der mindestens einen Reaktionsstufe, welcher aus zurückgeführten restlichen Oligomerisat und dem Frischzulauf des Einsatzgemisches besteht, mindestens 1 Gew.-%, bezogen auf die Gesamtzusammensetzung des Zulaufs, beträgt. Dies ist in Anspruch 1 wiedergegeben. Bevorzugte Ausgestaltungen des Verfahrens sind in den Unteransprüchen angegeben.

Das erfindungsgemäße Verfahren betrifft demnach ein an sich bekanntes Verfahren zur Oligomerisierung von C3- bis C5-Olefinen, vorzugsweise C4-Olefinen, welches durch die Rückführung bzw. Zuführung erhöhter Mengen an Oligomeren (erhöht im Vergleich zu normalen Oligomerisierungsverfahren) inhibiert wird. Die erfindungsgemäße Inhibierung wird jedoch nur dann durchgeführt, wenn ein vorher bestimmter Grenzwert eines Verfahrens- oder Anlagenparameters überschritten wird, wodurch angezeigt wird, dass in einem oder mehreren Reaktoren zumindest lokal (bezogen auf den jeweiligen Reaktor) eine zu hohe Reaktionsrate vorliegt. Der Verfahrens- oder Anlagenparameter, der bei Überschreiten eines vorher bestimmten Grenzwerts die zu hohe Reaktionsrate anzeigt ist der Temperaturanstieg (Differenz zwischen der Temperatur des Reaktoraustrags und der Temperatur des Reaktorzulaufs).

Die Wahl des geeignetsten Parameters zur Überwachung der Reaktionsrate ist von verschiedenen Faktoren, beispielsweise dem Aufbau der Anlage oder der Temperaturführung der Reaktoren abhängig. Hinsichtlich der Temperaturführung können die in dem erfindungsgemäßen Verfahren eingesetzten Reaktoren isotherm oder adiabatisch betrieben werden. Die Reaktoren in einer oder in allen Reaktionsstufen können dabei auf die gleiche Weise betrieben werden. Es ist aber auch möglich, dass in dem erfindungsgemäßen Verfahren sowohl isotherm als auch adiabatisch betriebene Reaktoren in einer oder allen Reaktionsstufen vorhanden sind. Adiabatisch betrieben heißt im Sinne der vorliegenden Erfindung, dass der Reaktor nicht isotherm betrieben wird, sondern ein Temperaturanstieg von > 10K zugelassen wird.

In einer Ausführungsform, bei der die Reaktionsstufe oder mindestens eine der Reaktionsstufen mindestens einen adiabatisch betriebenen Reaktor umfasst, wird der Temperaturanstieg überwacht, wobei sich als Grenzwert ein Temperaturanstieg von maximal 40 K ansetzen lässt. Oberhalb ist insbesondere bei Temperaturen im Reaktorzulauf von 60 °C oder mehr temperaturbedingt mit zumindest lokal erhöhten Reaktionsraten zu rechnen. Zudem kann die Temperatur der dem Reaktor entnommenen Produktmischung für die nachgeschalteten Verfahrensschritte zu hoch sein. Die maximale Temperatur innerhalb des Reaktors oder des Reaktorzulaufs bei dem vorhandenen Druck ist demnach vorzugsweise kleiner als die Siedetemperatur der eingesetzten Einsatzolefine. Die entsprechenden Temperatur-Druck-Paare für Siedepunkte der einsetzbaren Kohlenwasserstoffgemische sind dem Fachmann geläufig.

Demzufolge findet grundsätzlich zunächst ein klassisches Oligomerisierungsverfahren statt, bei dem ein Parameter überwacht wird und bei dem bei Überschreiten eines vorher bestimmten Grenzwerts des Parameters das erfindungsgemäße Verfahren zur Inhibierung dergestalt durchgeführt wird, dass die Menge an Oligomeren im Zulaufstrom zum Reaktor auf mindestens 1 Gew.-% eingestellt wird, um die gewünschte Inhibierung zu erreichen. Ist die Inhibierung erfolgreich, kann die Menge an Oligomeren im Zulaufstrom zum Reaktor auf den vorherigen Wert eingestellt werden bzw. die erfindungsgemäße Inhibierung beendet werden. Dies kann manuell oder automatisiert, beispielsweise Computer-gesteuert erfolgen. Die Einstellung der Menge an Oligomeren im Zulaufstrom auf mindestens 1 Gew.-% erfolgt dabei vorzugsweise nur so lange bis der Parameter wieder unter dem vorher bestimmten Grenzwert liegt, also nur temporär. Danach wird das Verfahren wieder unter den üblichen Produktionsbedingungen weitergeführt.

Der Gegenstand der vorliegenden Erfindung ist deshalb ein Verfahren zur Oligomerisierung von C3- bis C5-Olefinen, vorzugsweise C4-Olefinen, wobei die Oligomerisierung mit einem Einsatzgemisch, welches die C3- bis C5-Olefine, vorzugsweise C4-Olefine, enthält, unter Verwendung eines Oligomerisierungskatalysators in mindestens einer Reaktionsstufe unter Erhalt eines Oligomerisats erfolgt, wobei die gebildeten Oligomere in mindestens einer nachgeschalteten Destillationskolonne zumindest teilweise vom restlichen Oligomerisat, welches zumindest teilweise zum mindestens einen Reaktor zurückgeführt wird, abgetrennt werden, dadurch gekennzeichnet, dass

die Reaktionsstufe oder mindestens eine der Reaktionsstufen mindestens einen adiabatisch betriebenen Reaktor umfasst, wobei in dem adiabatisch betriebenen Reaktor zumindest zeitweise ein Temperaturanstieg, d. h. die Differenz zwischen der Temperatur des Reaktoraustrags und der Temperatur des Reaktorzulaufs (T im Reaktoraustrag - T im Reaktorzulauf), von 40 K oder mehr vorliegt, und dass

der Temperaturanstieg überwacht wird und bei Überschreiten eines TemperaturanstiegGrenzwerts von 40 K der Gehalt an Oligomeren im Zulauf zum mindestens einen Reaktor der mindestens einen Reaktionsstufe, welcher aus zurückgeführten restlichen Oligomerisat und dem Frischzulauf des Einsatzgemisches besteht, auf 1 Gew.-% bis 10 Gew.-%, .-%, bevorzugt 1 Gew.-% bis 5 Gew.-%, besonders bevorzugt 1 Gew.-% bis 3 Gew.-%, bezogen auf die Gesamtzusammensetzung des Zulaufs, eingestellt wird.

Die Überwachung des Temperaturanstiegs erfolgt demnach insbesondere durch das Messen der Temperatur am Zulauf und am Ablauf des Reaktors. Geeignete Messvorrichtungen sind dem Fachmann bekannt. Aus den beiden Werten kann der Temperaturanstiegt dann durch einfach Subtraktion ermittelt werden. Die Überwachung kann kontinuierlich oder diskontinuierlich, d. h. durch Messen in bestimmten Zeitintervallen, beispielsweise im Abstand von Sekunden, Minuten oder Stunden, durchgeführt werden. Die Überwachung und die dann einzustellende Inhibierung erfolgt vorzugsweise computergesteuert.

Fällt der Temperaturanstieg auf unter 40 K ab, wird der Gehalt an Oligomeren im Zulauf zum mindestens einem Reaktor der mindestens einen Reaktionsstufe wieder auf den Wert eingestellt, der vor der Umstellung, also vor der Inhibierung, vorgelegen hat.

Die Einstellung des Gehalts an Oligomeren im Zulauf kann durch die Trenneffizienz der Destillationskolonne(n) erfolgen, d. h. werden weniger Oligomere in der Destillation abgetrennt, können mehr Oligomere in den oder die Reaktoren gefahren werden, um zur Inhibierung beizutragen. Vorzugsweise wird der Gehalt an Oligomeren dadurch eingestellt, dass man Oligomere ins Destillat (restliches Oligomerisat) der Destillationskolonne(n) sieden lässt und dann das mit Oligomeren "verunreinigte" Gemisch zurück in den oder die Reaktoren fährt. Dies entspricht einer künstlichen Verschlechterung der Reinheit des zurückgeführten restlichen Oligomerisats.

Der Begriff "Reaktionsstufe" meint im Sinne der vorliegenden Erfindung einen Anlagenabschnitt, der einen oder mehreren Reaktor(en) und eine oder mehrere dem Reaktor nachfolgende Destillationskolonne(n) umfasst. In einer bevorzugten Ausführungsform ist nur eine Destillationskolonne pro Reaktionsstufe vorhanden. In den Destillationskolonnen werden die gebildeten Oligomere von dem Oligomerisat (entspricht dem Ausgangsstrom aus dem Reaktor), welches neben den Oligomeren auch Alkane und nicht umgesetzte Olefine umfasst, getrennt. Über die Destillationskolonnen kann auch die Effizienz der Auftrennung eingestellt werden, um die jeweils zur ausreichenden Inhibierung benötigte Menge an Oligomeren zu dem oder den Reaktoren führen zu können. Übliche verfahrenstechnische Aggregate, die in den Reaktionsstufen eingebaut sein können, wie beispielsweise Vorwärmer für den Feed, Wärmetauscher oder ähnliches, werden hier nicht separat aufgeführt, sondern sind dem Fachmann geläufig.

Das erfindungsgemäße Verfahren umfasst mindestens eine Reaktionsstufe. Das Verfahren kann aber auch mindestens zwei Reaktionsstufen umfassen, wobei vorzugsweise nicht mehr als fünf Reaktionsstufen vorliegen. In einer bevorzugten Ausführungsform umfasst das Verfahren zur Oligomerisierung daher zwei, drei, vier oder fünf Reaktionsstufen. Jede dieser Reaktionsstufen umfasst unabhängig voneinander einen oder mehreren Reaktoren und eine oder mehrere nachfolgende Destillationskolonnen, um die gebildeten Oligomere von dem restlichen Ausgangsstrom aus dem Reaktor abzutrennen. Es ist aber auch denkbar, dass eine der Reaktionsstufen mehrere Reaktoren umfasst, während in einer vorherigen oder nachfolgenden Reaktionsstufe nur ein Reaktor vorhanden ist.

Bei der Verfahrensführung mit zwei oder mehr Reaktionsstufen werden die in dem Reaktor oder in den Reaktoren der ersten Reaktionsstufe gebildeten Oligomere in der Destillationskolonne der ersten Reaktionsstufe so vom restliche Oligomerisat abgetrennt, dass der Gehalt an Oligomeren im Zulauf mindestens 1 Gew.-%, bezogen auf die Gesamtzusammensetzung des Zulaufs, beträgt, wenn das restliche Oligomerisat zumindest teilweise mit dem Frischzulauf vermischt und zum ersten Reaktor der ersten Reaktionsstufe geführt wird. Eine Kontrolle der Gehalte kann beispielsweise mittels gaschromatographischen Methoden erfolgen. Das entsprechende restliche Oligomerisat wird teilweise zu dem oder zu einem der Reaktor(en) der gleichen Reaktionsstufe und teilweise zur nächsten Reaktionsstufe geführt. Die zumindest teilweise Weiterleitung des restlichen Oligomerisats an die nächste Reaktionsstufe entfällt bei der letzten Reaktionsstufe naturgemäß. Neben der Rückführung zum Reaktor der gleichen Reaktionsstufe und der Weiterleitung an die nächste Reaktionsstufe kann auch ein Teil des restlichen Oligomerisats entnommen werden, beispielsweise um zu verhindern, dass sich inerte Alkane im System anreichern.

Das erfindungsgemäße Verfahren kann ganz allgemein wie folgt durchgeführt werden, vorzugsweise wenn die Inhibierung der Oligomerisierung erforderlich ist: Ausgangspunkt ist die Bereitstellung eines Einsatzgemisches, welches C3- bis C5-Olefine, vorzugsweise C4-Olefine enthält. Das Einsatzgemisch wird zunächst in dem mindestens einen Reaktor der ersten Reaktionsstufe oligomerisiert und das erhaltene Oligomerisat zu einer Destillationskolonne geleitet, bei der die gebildeten Oligomere (vorzugsweise C6- bis C20-Olefine, besonders bevorzugt C8- bis C20-Olefine) als Sumpfprodukt von dem restlichen Oligomerisat, welches zumindest nicht umgesetzte Olefine und Alkane aus dem Einsatzgemisch enthält und welches als Kopfprodukt anfällt, getrennt. Je nach Reaktionsstufe wird das restliche Oligomerisat dann zumindest teilweise als Zulaufstrom zur jeweils nächsten Reaktionsstufe geleitet und teilweise zum Reaktor der gleichen Reaktionsstufe recycliert und vorher mit dem Frischzulauf aus frischen Einsatzgemisch kombiniert, wobei der Gehalt an Oligomeren im Zulauf zur jeweiligen Reaktionsstufe mindestens 1 Gew.-%, vorzugsweise 1 Gew.-% bis 10 Gew.-%, beträgt. In der letzten Reaktionsstufe kann das Oligomerisat teilweise zum Reaktor in dieser oder einer vorherigen Reaktionsstufe recycliert und teilweise aus dem Verfahren ausgeschleust werden. Wird das restliche Oligomerisat aus der letzten Reaktionsstufe aus dem hier beschriebenen Verfahren ausgeschleust, kann dies als Syntheserohstoff für weitere Verfahren (z. B. Hydroformylierung, C-Quelle für Lichtbogen bei der Acetylenherstellung), als Verbrennungsgas oder nach Vollhydrierung zu den Alkanen als Treibgas, als Kochgas o. ä. dienen.

Als Olefine für das erfindungsgemäße Verfahren werden C3- bis C5-Olefine, bevorzugt C4-Olefine oder darauf basierende Olefingemische, die auch Anteile an analogen Alkanen enthalten können, eingesetzt. Geeignete Olefine sind unter anderem $\alpha$-Olefine, n-Olefine und Cycloalkene, vorzugsweise n-Olefine. In einer bevorzugten Ausführungsform handelt es sich bei dem C4-Olefin um n-Buten.

Die Olefine werden üblicherweise nicht in reiner Form als Edukte eingesetzt, sondern in technisch verfügbaren Mischungen. Der in dieser Erfindung verwendete Begriff Einsatzgemisch ist deshalb so zu verstehend, dass er jegliche Art von Gemischen betrifft, die die entsprechenden zu oligomerisierenden Olefine in einer Menge enthalten, die es ermöglicht, die Oligomerisierung wirtschaftlich durchzuführen. Bevorzugt enthalten die erfindungsgemäß verwendeten Einsatzgemische praktisch keine weiteren ungesättigten Verbindungen und mehrfach ungesättigte Verbindungen wie Diene oder Acetylenderivate. Vorzugsweise werden Einsatzgemische eingesetzt, die weniger als 5 Gew.-%, insbesondere weniger als 2 Gew.-% an verzweigten Olefinen bezogen auf den Olefinanteil enthalten.

Propylen (C3) wird großtechnisch durch Spaltung von Naphtha hergestellt und ist eine Grundchemikalie, die leicht verfügbar ist. C5-Olefine sind in Leichtbenzinfraktionen aus Raffinerien oder Crackern enthalten. Technische Gemische, die lineare C4-Olefine enthalten, sind Leichtbenzinfraktionen aus Raffinerien, C4-Fraktionen aus FC- oder Steamcrackern, Gemische aus Fischer-Tropsch-Synthesen, Gemische aus der Dehydrierung von Butanen und durch Metathese oder aus anderen technischen Prozessen entstandene Gemische. Beispielsweise können für das erfindungsgemäße Verfahren geeignete Gemische linearer Butene aus der C4-Fraktion eines Steamcrackers gewonnen werden. Dabei wird im ersten Schritt Butadien entfernt.

Dies geschieht entweder durch Extraktion(sdestillation) des Butadiens oder dessen Selektivhydrierung. In beiden Fällen wird ein praktisch butadienfreier C4-Schnitt erhalten, das sogenannte Raffinat I. Im zweiten Schritt wird Isobuten aus dem $C_4$-Strom entfernt, z. B. durch Herstellung von MTBE durch Umsetzung mit Methanol. Der jetzt isobutenfreie und butadienfreie C4-Schnitt, das sogenannte Raffinat II, enthält die linearen Butene und gegebenenfalls Butane. Wird daraus auch noch zumindest ein Teil des enthaltenen 1-Butens abgetrennt, erhält man das sogenannte Raffinat III.

In einer bevorzugten Ausführungsform werden im erfindungsgemäßen Verfahren C4-olefinhaltige Stoffströme als Einsatzgemisch zugeführt. Geeignete C4-olefinhaltige Stoffströme sind insbesondere das Raffinat I, das Raffinat II und das Raffinat III.

Als Reaktor für die jeweiligen Reaktionsstufen können alle dem Fachmann bekannten Reaktoren eingesetzt werden, die für die Oligomerisierung geeignet sind, bspw. Rohrreaktoren, Rohrbündelreaktoren, Settler-Riser-Reaktoren, Slurry-Reaktoren. Bevorzugt sind Rohrreaktoren und/oder Rohrbündelreaktoren. Sofern eine Reaktionsstufe mehrere Reaktoren aufweist, können die Reaktoren gleich oder verschieden voneinander sein. Die Reaktoren in einer Reaktionsstufe können auch in ihrem Aufbau oder ihrer Ausgestaltung variieren. Der erste Reaktor in einer Reaktionsstufe kann beispielsweise ein größeres Volumen aufweisen als der nachfolgende Reaktor in der gleichen Reaktionsstufe. Ebenso ist es möglich, dass die Reaktoren in den einzelnen Reaktionsstufen, sofern mehrere Reaktionsstufen vorliegen, gleich oder verschieden voneinander sein. Auch dabei ist es möglich, dass die Reaktoren in den einzelnen Reaktionsstufen in ihrem Aufbau oder ihrer Ausgestaltung unterschiedlich sind. Der Reaktor in der ersten Reaktionsstufe kann beispielsweise ein größeres Volumen aufweisen als ein oder alle Reaktoren in den nachfolgenden Reaktionsstufen.

Der eine oder die Reaktoren der einzelnen Reaktionsstufen enthalten jeweils einen heterogenen Oligomerisierungskatalysator zur Durchführung der Oligomerisierung. Der heterogene Oligomerisierungskatalysator liegt dabei insbesondere in Form eines Granulats, eines Extrudats oder in Tablettenform vor.

Die heterogenen Oligomerisierungskatalysatoren in den einzelnen Reaktoren der Reaktionsstufen können jeweils unabhängig voneinander aus Übergangsmetall-haltigen Oligomerisierungskatalysatoren ausgewählt werden. Die Übergangsmetalle bzw. die entsprechend eingesetzten Übergangsmetallverbindungen sind dabei vorzugsweise auf einem Trägermaterial, enthaltend Aluminiumoxid, Siliciumdioxid oder Alumosilicat, vorzugsweise einem Alumosilicat-Trägermaterial, angeordnet. Als Übergangsmetallverbindungen für die erfindungsgemäße eingesetzten Oligomerisierungskatalysatoren eignen sich insbesondere Verbindungen von Nickel, Cobalt, Chrom, Titan und Tantal. Bevorzugt sind Nickel- und Cobalt-Verbindungen, besonders bevorzugt sind Nickel-Verbindungen.

In einer bevorzugten Ausführungsform umfasst der erfindungsgemäße Oligomerisierungskatalysator eine Nickelverbindung, vorzugsweise Nickeloxid, und ein Trägermaterial, welches Aluminiumoxid, Siliciumdioxid oder Alumosilicat, vorzugsweise Alumosilicat, umfasst daraus besteht, eingesetzt werden. Das Trägermaterial ist vorzugsweise ein amorphes, mesoporöses Alumosilicat, ein kristallines, mikroporöses Alumosilicat oder ein Alumosilicat, welches amorphe und kristalline Phasen aufweist. Mit "amorph" im Sinne der vorliegenden Erfindung ist die Eigenschaft eines Feststoffes gemeint, die daraus folgt, dass der Feststoff keine Kristallstruktur, d. h. keine Fernordnung, aufweist. Im Sinne der vorliegenden Erfindung ist aber nicht auszuschließen, dass das amorphe Alumosilicat kleine kristalline Domänen aufweist.

Erfindungsgemäß weiterhin bevorzugt weist der Oligomerisierungskatalysator eine Zusammensetzung von 15 bis 40 Gew.-%, vorzugsweise 15 bis 30 Gew.-% NiO, 5 bis 30 Gew.-% $Al_2O_3$, 55 bis 80 Gew.-% $SiO_2$ und 0,01 bis 2,5 Gew.-%, vorzugsweise 0,05 bis 2 Gew.-% eines Alkalimetalloxids, vorzugsweise Natriumoxid, auf. Die Angaben beziehen sich auf eine Gesamtzusammensetzung von 100 Gew.-%. In einer besonders bevorzugten Ausführungsform der vorliegenden Erfindung ist der Oligomerisierungskatalysator im Wesentlichen frei von Titandioxid und/oder Zirkoniumdioxid, insbesondere enthält der Oligomerisierungskatalysator weniger als 0,5 Gew.-%, bevorzugt weniger als 0,1 Gew.-%, besonders bevorzugt weniger als 0,01 Gew.-% Titandioxid und/oder Zirkoniumdioxid in seiner Gesamtzusammensetzung.

Der Oligomerisierungskatalysator weist vorzugsweise eine spezifische Oberfläche (berechnet nach BET) von 150 bis 700 $m^2/g$, weiterhin bevorzugt von 190 bis 600 $m^2/g$, besonders bevorzugt von 220 bis 550 $m^2/g$ aufweisen. Die BET-Oberfläche wird mittels Stickstoff-Physisorption gemäß DIN-ISO 9277 (Stand: 2014-01) gemessen.

Sollten mehrere Reaktoren in einer Reaktionsstufe oder in mehreren Reaktionsstufen vorhanden sein, liegen naturgemäß auch mehrere Oligomerisierungskatalysatoren vor. Die in den einzelnen Reaktoren in den Reaktionsstufen vorhandenen Oligomerisierungskatalysatoren können jeweils unabhängig voneinander aus den vorgenannten Substanzen ausgewählt werden. Die einzelnen Oligomerisierungskatalysatoren in den Reaktoren sind dabei nicht immer exakt identisch, sondern unterscheiden sich in der Zusammensetzung voneinander, möglicherweise auch nur in geringem Umfang. Dies liegt auch daran, dass selbst wenn zum Zeitpunkt der ersten Inbetriebnahme des erfindungsgemäßen Verfahrens jeder Reaktor eine völlig identische Katalysatorzusammensetzung enthält, sich diese Zusammensetzung während des Betriebs mit der Zeit durch verschiedenste Effekte im Laufe der Jahre ändern.

Die Herstellung eines Oligomerisierungskatalysators kann nach den bekannten Verfahren des Imprägnierens, wobei das Trägermaterial mit einer Lösung einer Übergangsmetallverbindung, insbesondere eine Nickelverbindung, beaufschlagt und danach kalziniert wird, oder Co-Fällung, bei der die gesamte Katalysatorzusammensetzung aus einer einzigen, zumeist wässrigen Lösung ausgefällt wird, erfolgen. Der Oligomerisierungskatalysator kann auch nach anderen, dem Fachmann geläufigen, Verfahren hergestellt werden.

Die Oligomerisierung kann in jeder der vorhandenen Reaktionsstufen bei einer Temperatur im Bereich von 50 bis 200 °C, vorzugsweise 60 bis 180 °C, bevorzugt im Bereich von 60 bis 130°C durchgeführt wird. Der Temperaturanstieg im Reaktor kann erfindungsgemäß maximal 40 K betragen. Der Temperaturanstieg ist definiert als die Differenz zwischen der Temperatur des Reaktoraustrags und der Temperatur des Reaktorzulaufs (T im Reaktoraustrag - T im Reaktorzulauf). Der Druck kann jeder der vorhandenen Reaktionsstufen von 10 bis 70 bar, bevorzugt von 20 bis 55 bar betragen. In einer bevorzugten Ausführungsform der vorliegenden Erfindung wird die Oligomerisierung in jeder Reaktionsstufe in flüssiger Phase durchgeführt. Sofern die Oligomerisierung in flüssiger Phase erfolgen soll, müssen die Parameter Druck und Temperatur hierfür so gewählt werden, dass der Eduktstrom (die eingesetzten Olefine oder Olefingemische) in flüssiger Phase vorliegt.

Die gewichtbasierten Raumgeschwindigkeiten (Reaktandmasse pro Katalysatormasse pro Zeit; weight hourly space velocity (WHSV)) befinden sich im Bereich zwischen 1 g Reaktand pro g Katalysator und pro h (= 1 h$^{-1}$) und 190 h$^{-1}$, vorzugsweise zwischen 2 h$^{-1}$ und 35 h$^{-1}$, besonders bevorzugt zwischen 3 h$^{-1}$ und 25 h$^{-1}$.

Die Linearität eines Oligomerisierungsprodukts bzw. von den entstandenen Dimeren wird durch den ISO-Index beschrieben und stellt einen Wert für die mittlere Anzahl an Methylverzweigungen im Dimer dar. So tragen (für Buten als Edukt) z.B. n-Oktene mit 0, Methylheptene mit 1 und Dimethylhexene mit 2 zum ISO-Index einer C8-Fraktion bei. Je niedriger der ISO-Index ist, umso linearer sind die Moleküle in der jeweiligen Fraktion aufgebaut. Der ISO-Index errechnet sich nach folgender allgemeiner Formel, wobei sich der Anteil der einzelnen Dimerfraktionen auf die Gesamt-dimer-Fraktion bezieht:

$$\frac{\text{(einfach verzweigte Dimere (Gew.-\%)} + 2 \times \text{zweifach verzweigte Dimere (Gew.-\%))}}{100}$$

Demnach besitzt ein Dimerengemisch mit einem ISO-Index von 1,0 im Mittel genau eine Methylverzweigung pro Dimerenmolekül.

Der ISO-Index des Produkts aus dem erfindungsgemäßen Oligomerisierungsverfahren beträgt vorzugsweise 0,8 bis 1,2, besonders bevorzugt 0,8 bis 1,15.

Die nach dem erfindungsgemäßen Verfahren hergestellten Oligomere werden unter anderem zur Herstellung von Aldehyden, Alkoholen und Carbonsäuren genutzt. So ergibt beispielsweise das Dimerisat aus linearen Butenen durch Hydroformylierung ein Nonanalgemisch. Dieses liefert entweder durch Oxidation die entsprechenden Carbonsäuren oder durch Hydrierung ein C9-Alkoholgemisch. Das C9-Säuregemisch kann zur Herstellung von Schmiermitteln oder Sikkative verwendet werden. Das C9-Alkoholgemisch ist Vorstufe für die Herstellung von Weichmachern, insbesondere von Di-Nonyl-phthalaten oder DINCH.

Beispiele

Beispiel 1 (nicht erfindungsgemäß):

Die Oligomerisierung wurde in einem weitgehend isotherm betriebenen Rohreaktor mit folgenden Maßen nachgestellt: Länge 2.0 m, Innendurchmesser 6 mm. Der Reaktor war zur Thermostatisierung in einen Thermostaten eingehängt. Als Wärmeträger diente das Produkt Marlotherm der Firma Sasol. Als Katalysator wurden 12,6 g eines Materials verwendet, welches gemäß Beispiel 1 der WO2011/000697A1 hergestellt und nach Beispiel 4 derselben Veröffentlichung nachbehandelt worden war.

Die Reaktion wurde bei einem Absolutdruck von 30 bar und einer Temperatur von 100°C in der Flüssigphase durchgeführt. Als Frischzulauf zum Reaktor wurden 118,5 g/h eines C4-Kohlenwasserstoffgemisches verwendet, enthaltend folgende Komponenten:

1-Buten 22,2 Gew.-%
2-Buten 57,9 Gew.-%
Isobuten 0,6 Gew.-%
Butane 18,2 Gew.-%
C8-Olefine 1,1 Gew.-%

Es wurde ein Umsatz an C4-Olefinen von 41,1 % erzielt, wobei folgende Oligomerisatverteilung im Reaktoraustrag erzielt wurden:

C8-Olefine 83,9 %
C12-Olefine 12,4
%
C16+-Olefine 3,8
%

Dies entspricht einer Produktmenge von 32,0 g/h an C8-Olefinen und einer Gesamtoligomerisatmenge von 38,5 g/h.

Beispiel 2 (nicht erfindungsgemäß):

Die Oligomerisierung wurde in einem weitgehend isotherm betriebenen Rohreaktor mit folgenden Maßen nachgestellt: Länge 2.0 m, Innendurchmesser 6 mm. Der Reaktor war zur Thermostatisierung in einen Thermostaten eingehängt. Als Wärmeträger diente das Produkt Marlotherm der Firma Sasol. Als Katalysator wurden 12,6 g eines Materials verwendet, welches gemäß Beispiel 1 der WO2011/000697A1 hergestellt und nach Beispiel 4 derselben Veröffentlichung nachbehandelt worden war.
Die Reaktion wurde bei einem Absolutdruck von 30 bar und einer Temperatur von 100°C in der Flüssigphase durchgeführt. Als Frischzulauf zum Reaktor wurden 118,5 g/h eines C4-Kohlenwasserstoffgemisches verwendet, enthaltend folgende Komponenten:

1-Buten 21,4 Gew.-%
2-Buten 57,7 Gew.-%
Isobuten 0,5 Gew.-%
Butane 17,3 Gew.-%
C8-Olefine 3,1 Gew.-
%

Es wurde ein Umsatz an C4-Olefinen von 38,0 % erzielt, wobei folgende Oligomerisatverteilung im Reaktoraustrag erzielt wurden:

C8-Olefine 83,4 %
C12-Olefine 13,0
%
C16+-Olefine 3,6
%

Dies entspricht einer Produktmenge von 29,6 g/h an C8-Olefinen und einer Gesamtoligomerisatmenge von 35,6 g/h.

Beispiel 3 (nicht erfindungsgemäß):

Die Oligomerisierung wurde in einem weitgehend isotherm betriebenen Rohreaktor mit folgenden Maßen nachgestellt: Länge 2.0 m, Innendurchmesser 6 mm. Der Reaktor war zur Thermostatisierung in einen Thermostaten eingehängt. Als Wärmeträger diente das Produkt Marlotherm der Firma Sasol. Als Katalysator wurden 12,6 g eines Materials verwendet, welches gemäß Beispiel 1 der WO2011/000697A1 hergestellt und nach Beispiel 4 derselben Veröffentlichung nachbehandelt worden war.
Die Reaktion wurde bei einem Absolutdruck von 30 bar und einer Temperatur von 80°C in der Flüssigphase durchgeführt. Als Frischzulauf zum Reaktor wurde 1 kg/h eines C4-Kohlenwasserstoffgemisches verwendet, enthaltend folgende Komponenten:

1-Buten      22,7 Gew.-%
2-Buten      58,4 Gew.-%
Isobuten     0,7 Gew.-%
Butane       18,1 Gew.-%

(fortgesetzt)

| | |
|---|---|
| C8-Olefine | 0,1 Gew.-% |

Es wurde ein Umsatz an C4-Olefinen von 43,9 % erzielt, wobei folgende Oligomerisatverteilung im Reaktoraustrag erzielt wurden:

| | |
|---|---|
| C8-Olefine | 83,9 % |
| C12-Olefine | 12,5 % |
| C16+-Olefine | 3,7 % |

Dies entspricht einer Produktmenge von 34,4 g/h an C8-Olefinen und einer Gesamtoligomerisatmenge von 41,3 g/h.

Beispiel 4 (nicht erfindungsgemäß):

Die Oligomerisierung wurde in einem weitgehend adiabat betriebenen Rohreaktor ohne Thermostatisierung mit folgenden Maßen nachgestellt: Länge 2.0 m, Innendurchmesser 20,5 mm. Als Katalysator wurden 300 g eines Materials verwendet, welches gemäß Beispiel 1 der WO2011/000697A1 hergestellt und nach Beispiel 4 derselben Veröffentlichung nachbehandelt worden war.
Die Reaktion wurde bei einem Absolutdruck von 30 bar und einer Reaktorzulauftemperatur von 90°C in der Flüssigphase durchgeführt. Als Frischzulauf zum Reaktor wurden 1,75 kg/h eines C4-Kohlenwasserstoffgemisches verwendet, enthaltend folgende Komponenten:

| | |
|---|---|
| 1-Buten | 35,8 Gew.-% |
| 2-Buten | 42,4 Gew.-% |
| Isobuten | 0,9 Gew.-% |
| Butane | 20,9 Gew.-% |
| C8-Olefine | 0,0 Gew.-% |

Es wurde ein Umsatz an C4-Olefinen von 31,7 % erzielt, wobei folgende Oligomerisatverteilung im Reaktoraustrag erzielt wurden:

| | |
|---|---|
| C8-Olefine | 85,1 % |
| C12-Olefine | 12,6 % |
| C16+-Olefine | 2,3 % |

Die maximale Temperatur des Reaktoraustrags betrug 130°C.

Beispiel 5 (nicht erfindungsgemäß):

Die Bedingungen und der Reaktor entsprachen denen des Beispiels 4. Es wurde ein C4-Gemisch eingesetzt, dass folgende Zusammensetzung aufwies:

| | |
|---|---|
| 1-Buten | 36,0 Gew.-% |
| 2-Buten | 43,8 Gew.-% |
| Isobuten | 0,7 Gew.-% |
| Butane | 19,3 Gew.-% |
| C8-Olefine | 0,2 Gew.-% (entspricht 3,5 g/h) |

Es wurde ein Umsatz an C4-Olefinen von 31,7 % erzielt, wobei folgende Oligomerisatverteilung im Reaktoraustrag erzielt wurden:

| | |
|---|---|
| C8-Olefine | 85,0 % |

(fortgesetzt)

| | |
|---|---|
| C12-Olefine | 12,6 % |
| C16+-Olefine | 2,4 % |

Die maximale Temperatur des Reaktoraustrags betrug 131 °C. Es konnte kein Umsatzrückgang gegenüber Beispiel 4 festgestellt werden. Der Temperaturanstieg ist weiterhin > 40 K.

Beispiel 6 (nicht erfindungsgemäß):

Die Bedingungen und der Reaktor entsprachen denen des Beispiels 4. Es wurde ein C4-Gemisch eingesetzt, dass folgende Zusammensetzung aufwies:

| | |
|---|---|
| 1-Buten | 36,1 Gew.-% |
| 2-Buten | 44,1 Gew.-% |
| Isobuten | 1,2 Gew.-% |
| Butane | 18,0 Gew.-% |
| C8-Olefine | 0,4 Gew.-% (entspricht 7 g/h) |

Es wurde ein Umsatz an C4-Olefinen von 30,1 % erzielt, wobei folgende Oligomerisatverteilung im Reaktoraustrag erzielt wurden:

| | |
|---|---|
| C8-Olefine | 85,0 % |
| C12-Olefine | 12,6 % |
| C16+-Olefine | 2,4 % |

Die maximale Temperatur des Reaktoraustrags betrug 128°C. Der Temperaturanstieg fiel nur geringfügig unter einen Temperaturanstieg von 40K und der Umsatz ging nur leicht zurück.

Beispiel 8 (erfindungsgemäß):

Die Oligomerisierung wurde in einem weitgehend adiabat betriebenen Rohreaktor mit folgenden Maßen nachgestellt: Länge 2.0 m, Innendurchmesser 20,5 mm.
Als Katalysator wurden 300 g eines Materials verwendet, welches gemäß Beispiel 1 der WO2011/000697A1 hergestellt und nach Beispiel 4 derselben Veröffentlichung nachbehandelt worden war.
Die Reaktion wurde bei einem Absolutdruck von 30 bar und einer Temperatur des Reaktorzulaufs von 75°C in der Flüssigphase durchgeführt. Als Frischzulauf zum Reaktor wurden 1,8 kg/h eines C4-Kohlenwasserstoffgemisches verwendet, enthaltend folgende Komponenten:

| | |
|---|---|
| 1-Buten | 42,5 Gew.-% |
| 2-Buten | 11,8 Gew.-% |
| Isobuten | 0,1 Gew.-% |
| Butane | 45,2 Gew.-% |
| C8-Olefine | 0,0 Gew.-% |

Es wurde ein Umsatz an C4-Olefinen von 26,3 % erzielt, wobei folgende Oligomerisatverteilung im Reaktoraustrag erzielt wurden:

| | |
|---|---|
| C8-Olefine | 87,1 % |
| C12-Olefine | 12,1 % |
| C16+-Olefine | 0,8 % |

Die maximale Temperatur des Reaktoraustrags betrug 120°C. Dies entspricht einem Temperaturanstieg von 45 K.

Es wurde mit der C8-Dosierung von 1,1 Gew.-% zusätzlich zum C4-Zulauf gestartet. Es wurden demnach 20 g/h C8 zudosiert. Eine Inhibierung trat unverzüglich ein. Die Dosierung wurde so lange durchgeführt bis der Temperaturanstieg wieder unter den Grenzwert von 40K gefallen war. Zu diesem Zeitpunkt wurde ein Umsatz von 20,7 % erzielt, wobei folgende Oligomerisatverteilung im Reaktoraustrag erzielt wurden:

| | |
|---|---|
| C8-Olefine | 89,2 % |
| C12-Olefine | 9,9 % |
| C16+-Olefine | 0,9 % |

Es konnte gezeigt werden, dass eine Erhöhung der C8-Olefingehaltes im Zulauf zum Reaktor über 1 Gew.-% hinaus zu einer Verringerung des Umsatzes und auch Gesamtoligomerisat führt, wodurch die Oligomerisierungsreaktion effektiv und gewollt inhibiert werden kann.

**Patentansprüche**

1. Verfahren zur Oligomerisierung von C3- bis C5-Olefinen, wobei die Oligomerisierung mit einem Einsatzgemisch, welches die C3- bis C5-Olefine enthält, unter Verwendung eines Oligomerisierungskatalysators in mindestens einer Reaktionsstufe unter Erhalt eines Oligomerisats erfolgt, wobei die gebildeten Oligomere in mindestens einer nachgeschalteten Destillationskolonne zumindest teilweise vom restlichen Oligomerisat, welches zumindest teilweise zum mindestens einen Reaktor zurückgeführt wird, abgetrennt werden, **dadurch gekennzeichnet, dass**

   die Reaktionsstufe oder mindestens eine der Reaktionsstufen mindestens einen adiabatisch betriebenen Reaktor umfasst, wobei in dem adiabatisch betriebenen Reaktor zumindest zeitweise ein Temperaturanstieg, d. h. die Differenz zwischen der Temperatur des Reaktoraustrags und der Temperatur des Reaktorzulaufs, von 40 K oder mehr vorliegt, und dass
   der Temperaturanstieg überwacht wird und bei Überschreiten eines Temperaturanstieggrenzwerts von 40 K der Gehalt an Oligomeren im Zulauf zum mindestens einen Reaktor der mindestens einen Reaktionsstufe, welcher aus zurückgeführten restlichen Oligomerisat und dem Frischzulauf des Einsatzgemisches besteht, auf 1 Gew.-% bis 10 Gew.-%, bezogen auf die Gesamtzusammensetzung des Zulaufs, eingestellt wird.

2. Verfahren nach Anspruch 1, wobei der heterogene Oligomerisierungskatalysator ein Übergangsmetall-haltiger Oligomerisierungskatalysator ist, der eine Übergangsmetallverbindung und ein Trägermaterial, vorzugsweise ein Alumosilicat-Trägermaterial umfasst.

3. Verfahren nach Anspruch 2, wobei der heterogene Oligomerisierungskatalysator eine Zusammensetzung von 15 bis 40 Gew.-% NiO, 5 bis 30 Gew.-% $Al_2O_3$, 55 bis 80 Gew.-% SiOz und 0,01 bis 2,5 Gew.-% eines Alkalimetalloxids aufweist.

4. Verfahren nach einem der Ansprüche 1 bis 3, wobei die Oligomerisierung in jeder der vorhandenen Reaktionsstufen bei einer Temperatur im Bereich von 50 bis 200 °C, vorzugsweise 60 bis 180 °C, besonders bevorzugt im Bereich von 60 bis 130°C durchgeführt wird.

5. Verfahren nach einem der Ansprüche 1 bis 4, wobei der Druck bei der Oligomerisierung in jeder der vorhandenen Reaktionsstufen von 10 bis 70 bar, bevorzugt von 20 bis 55 bar beträgt.

6. Verfahren nach einem der Ansprüche 1 bis 5, wobei bei dem Verfahren C4-Olefine eingesetzt werden.

**Claims**

1. Process for the oligomerization of C3- to C5-olefins, wherein the oligomerization is carried out with a feed mixture comprising the C3- to C5-olefins, using an oligomerization catalyst in at least one reaction stage to obtain an oligomerizate, wherein the oligomers formed are separated at least partially in at least one downstream distillation column from the residual oligomerizate, which is at least partially recycled to the at least one reactor, **characterized in that**

the reaction stage or at least one of the reaction stages comprises at least one adiabatically operated reactor, wherein in the adiabatically operated reactor a temperature increase, i.e. the difference between the temperature of the reactor discharge and the temperature of the reactor feed, is at least temporarily 40 K or more, and **in that** the temperature increase is monitored and, when the temperature increase exceeds a threshold value of 40 K, the oligomer content in the feed to the at least one reactor of the at least one reaction stage, which consists of recycled residual oligomerizate and the fresh feed of the feed mixture, is adjusted to 1% by weight to 10% by weight, based on the total composition of the feed.

2. Process according to Claim 1, wherein the heterogeneous oligomerization catalyst is a transition metal-containing oligomerization catalyst comprising a transition metal compound and a support material, preferably an aluminosilicate support material.

3. Process according to Claim 2, wherein the heterogeneous oligomerization catalyst has a composition of 15 to 40% by weight NiO, 5 to 30% by weight $Al_2O_3$, 55 to 80% by weight $SiO_2$ and 0.01 to 2.5% by weight of an alkali metal oxide.

4. Process according to any of Claims 1 to 3, wherein the oligomerization in each of the reaction stages present is carried out at a temperature in the range of 50 to 200°C, preferably 60 to 180°C, particularly preferably in the range of 60 to 130°C.

5. Process according to any of Claims 1 to 4, wherein the pressure in the oligomerization in each of the reaction stages present is from 10 to 70 bar, preferably from 20 to 55 bar.

6. Process according to any of Claims 1 to 5, wherein C4-olefins are used in the process.

**Revendications**

1. Procédé d'oligomérisation d'oléfines en C3 à C5, l'oligomérisation ayant lieu avec un mélange de charge qui contient les oléfines en C3 à C5, par utilisation d'un catalyseur d'oligomérisation dans au moins un étage de réaction avec obtention d'un oligomère, les oligomères formés étant dans au moins une colonne de distillation en aval séparés au moins partiellement de l'oligomère restant, qui est au moins partiellement renvoyé à l'au moins un réacteur, **caractérisé en ce que** l'étage de réaction ou au moins l'un des étages de réaction comprend au moins un réacteur adiabatique, une élévation de la température, c'est-à-dire la différence entre la température du produit du réacteur et la température de la charge d'alimentation du réacteur, étant au moins par intermittence de 40 K ou plus dans le réacteur adiabatique, et **en ce que**
l'élévation de température est surveillée et, en cas de dépassement d'une limite d'élévation de température de 40 K, la teneur en oligomères de la charge d'alimentation d'au moins un réacteur de l'au moins un étage de réaction, qui est constituée de l'oligomère résiduel renvoyé et de la charge fraîche du mélange de charge, est ajustée à 1 % en poids à 10 % en poids par rapport à la composition totale de la charge d'alimentation.

2. Procédé selon la revendication 1, dans lequel le catalyseur hétérogène d'oligomérisation est un catalyseur d'oligomérisation contenant un métal de transition, qui comprend un composé d'un métal de transition et un matériau support, de préférence un matériau support aluminosilicate.

3. Procédé selon la revendication 2, dans lequel le catalyseur hétérogène d'oligomérisation présente une composition de 15 à 40 % en poids de NiO, de 5 à 30 % en poids d'$Al_2O_3$, de 55 à 80 % en poids de $SiO_2$ et de 0,01 à 2,5 % en poids d'un oxyde d'un métal alcalin.

4. Procédé selon l'une des revendications 1 à 3, dans lequel l'oligomère, dans chacun des étages de réaction présents, est mis en œuvre à une température dans la plage de 50 à 200 °C, de préférence de 60 à 180 °C, d'une manière particulièrement préférée dans la plage de 60 à 130 °C.

5. Procédé selon l'une des revendications 1 à 4, dans lequel la pression, lors de l'oligomérisation dans chacun des étages de réaction présents, est de 10 à 70 bars, de préférence de 20 à 55 bar.

6. Procédé selon l'une des revendications 1 à 5, des oléfines en C4 étant utilisées dans le procédé.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014207034 A1
- EP 1457475 A2

- WO 2011000697 A1